# EUROPÄISCHE PATENTSCHRIFT

(11) **EP 3 630 279 B1**
(45) Veröffentlichungstag und Bekanntmachung des Hinweises auf die Patenterteilung: **10.11.2021**
(21) Anmeldenummer: 18723395.2
(22) Anmeldetag: 23.04.2018
(51) Int. Cl.: A61N 1/44, A61L 2/14, H05H 1/24

(54) **FLÄCHIGE AUFLAGEANORDNUNG AUSGEBILDET ZUR ERZEUGUNG EINES DIELEKTRISCH BEHINDERTEN PLASMAS**
FLAT PAD STRUCTURE CONFIGURED TO GENERATE A DIELECTRIC BARRIER PLASMA DISCHARGE
ENSEMBLE DE SUPPORT PLAT CONFIGURÉ POUR GÉNÉRER UNE DÉCHARGE À BARRIÈRE DIÉLECTRIQUE DE PLASMA

(30) Priorität: 31.05.2017 DE 102017111902
(43) Veröffentlichungstag der Anmeldung: 08.04.2020
(73) Patentinhaber: Cinogy GmbH, 37115 Duderstadt (DE)
(72) Erfinder: WANDKE, Dirk, 37308 Heilbad Heiligenstadt (DE); HAHNL, Mirko, 37339 Berlingerode (DE); STORCK, Karl-Otto, 37115 Duderstadt (DE); RICKE, Melanie, 37191 Katlenburg-Lindau (DE); TRUTWIG, Leonhard, 37115 Duderstadt (DE)
(74) Vertreter: Gramm, Lins & Partner Patent- und Rechtsanwälte PartGmbB
(86) Internationale Anmeldenummer: PCT/DE2018/100388
(87) Internationale Veröffentlichungsnummer: WO 2018/219383

(56) Entgegenhaltungen:
- EP-A1- 3 051 926
- DE-A1-102011 105 713
- DE-A1-102015 118 372
- KR-B1- 101 709 167
- US-A1- 2013 345 620
- US-A1- 2015 157 870
- US-A1- 2016 331 989

## Beschreibung

Die Erfindung betrifft eine flächige Auflageanordnung, ausgebildet zur Erzeugung eines dielektrisch behinderten Plasmas an einer Anlageseite der Auflageanordnung, mit wenigstens einer flächigen Elektrodenanordnung, die in ein flächiges Dielektrikum eingebettet, mit Hochspannungssignalen versorgbar und allseitig gegen einen unbehinderten Stromfluss abgeschirmt ist, wobei sich die Auflagenanordnung mit einer Breite in Längsrichtung erstreckt und wobei die Länge der Auflageanordnung in dieser Längsrichtung verkleinerbar ist.

Eine derartige flächige Auflageanordnung ist durch EP 2 723 447 B1 bekannt. Das Dielektrikum weist einen Zentralbereich auf, von dem aus ein spiralförmig, mehrere Windungen ausbildender Streifen den Randbereich der Auflageanordnung bildet. Um die wirksame Auflagefläche der Auflageanordnung auf der Anlageseite des Dielektrikums an eine Unterlage hinsichtlich der Größe anzupassen, kann der spiralförmig ausgebildete Streifen an einer geeigneten Stelle mit Hilfe eines Werkzeugs gekürzt werden, um so die Anlagefläche in der gewünschten Weise zu verkleinern. Auf diese Weise kann ein dielektrisch behindertes Plasmafeld in der benötigten Größe mittels der Auflageanordnung erzeugt werden und beispielsweise auf eine Hautfläche eines menschlichen oder tierischen Körpers einwirken. Dabei kann die Haut oder eine andere zu behandelnde Oberfläche als Gegenelektrode fungieren, wenn der die Oberfläche aufweisende Körper ausreichend leitfähig ist. Die Elektrode wird mit einer Hochspannung versorgt, die ausreicht, um das Plasma in einem Luftraum zwischen der Auflageanordnung und der zu behandelnden Oberfläche, insbesondere der Haut, zu erzeugen. Damit ein definierter Luftraum zustande kommt, wenn die Elektrode an der zu behandelnden Oberfläche anliegt, kann das Dielektrikum an seiner Anlagefläche mit einer Struktur in Form von Noppen oder eines Gitters o. ä. versehen sein, deren Oberseite zur Anlage an der zu behandelnden Fläche ausgebildet ist und zwischen den Anlagepunkten, -flächen oder -linien ausreichende Luftzwischenräume bildet, in denen die dielektrisch behinderte Plasmaentladung stattfinden kann.

Nach der Ablängung des Streifens, beispielsweise mit einer Schere, wird die Schnittkante mit einem isolierenden Kontaktelement übergriffen, das eine Kontaktierung mit dem die Elektrode bildenden elektrischen Leiter bewirkt, beispielsweise mittels Schneidkontakten. Über das Kontaktelement wird das extern generierte Hochspannungssignal auf die Elektrode übertragen. Die Ausbildung der Auflageanordnung mit dem spiralförmig mehr als eine ganze Windung bildenden Streifen erlaubt zwar eine Anpassung der wirksamen Anlagefläche an einen speziellen Einsatzfall, führt jedoch zu einer gewissen Instabilität der Anlageanordnung. Darüber hinaus stößt die Anpassbarkeit der Anlagefläche an Grenzen, wenn beispielsweise die Behandlung einer Wunde beabsichtigt ist, die nicht im Wesentlichen kreisförmig ausgebildet ist, sondern länglich.

US 2015/0157870 A1 offenbart eine flächige Auflageanordnung der eingangs erwähnten Art, bei der die Hochspannungselektroden kammförmig ineinander verschränkt ausgebildet sind. In einer nicht näher dargestellten Weise sollen die Elektroden abbrechbare Abschnitte aufweisen, sodass die Enden der Elektroden innerhalb der flexiblen Auflageanordnung verbleiben, wenn die Auflageanordnung in ihrer Größe zugeschnitten wird.

Aus DE 10 2011 105 713 A1 ist eine flexible flächige Elektrodenanordnung für eine dielektrisch behinderte Gasentladung, mit einem zentralen Bereich und einem Randbereich und mit einer ein Hochspannungspotential führenden flächigen Elektrode bekannt, die in einem flächigen eine Oberseite und eine Anlageseite bildenden Dielektrikum eingebettet ist und eine Anpassung der Wirkfläche der Elektrodenanordnung an die Größe einer zu behandelnden Oberfläche erlaubt.

Der vorliegenden Erfindung liegt daher die Aufgabe zu Grunde, eine flächige Auflageanordnung der bekannten Art hinsichtlich ihrer Stabilität und Anpassbarkeit an nicht runde Behandlungsflächen zu verbessern.

Zur Lösung dieser Aufgabe ist eine flächige Auflageanordnung der eingangs erwähnten Art dadurch gekennzeichnet, dass die Auflageanordnung in Längsrichtung mehrere gleich aufgebaute Abschnitte mit jeweils einem Dielektrikumsabschnitt in der Breite der Auflageanordnung und mit jeweils wenigstens einem Elektrodenabschnitt aufweist, wobei die Elektrodenabschnitte der Abschnitte in Längsrichtung aneinander anschließen und eine sich über die gesamte Länge erstreckende Elektrodenanordnung bilden, dass wenigstens ein Abschnitt von einem benachbarten Abschnitt zur Verkleinerung der Anlagefläche in Längsrichtung an einer quer zur Längsrichtung verlaufenden Soll-Trennlinie abtrennbar ist und dass an dem verbleibenden benachbarten Abschnitt die Soll-Trennlinie mit einem isolierenden Bauelement abgedeckt ist.

Erfindungsgemäß wird ein prinzipiell neuer Aufbau der flächigen Auflageanordnung vorgeschlagen, bei dem sich gleich aufgebaute Abschnitte mit einer Breite des Dielektrikums, die der Breite der Auflageanordnung - abgesehen von etwaigen Klebeansätzen - entspricht. Durch das mögliche Abtrennen von einem Abschnitt oder mehreren Abschnitten kann die Länge der Auflageanordnung an den jeweiligen Einsatzfall angepasst werden. Die Breite bleibt dabei unverändert. Durch das Abtrennen eines Abschnitts entlang der Soll-Trennlinie ragt in dem verbleibenden Abschnitt, der nunmehr ein Randabschnitt ist, der zugehörige Elektrodenabschnitt in die Trennfläche an der Soll-Trennlinie hinein und könnte unmittelbar kontaktiert werden, beispielsweise durch eine die Plasmabehandlung durchführende Person. Dies wird dadurch vermieden, dass die erfindungsgemäße Auflageanordnung die Soll-Trennlinie in dem verbleibenden Abschnitt mit einem isolierenden Bauelement abdeckt, das sich somit ebenfalls über die Breite des Dielektrikums, also über die Breite der Anlagefläche der Auflageanordnung, erstreckt.

Die erfindungsgemäße Auflageanordnung ist insbesondere für die Behandlung von Wunden, insbesondere Operationswunden, von Bedeutung. Die Auflageanordnung kann als Wundabdeckung auf die Haut aufgelegt und mit entsprechend angeformten Klebelaschen klebend befestigt werden. In sinnvollen zeitlichen Abständen kann dann die Plasmabehandlung erfolgen, um Keime im Wundbereich abzutöten und/oder die Mikrozirkulation im Gewebe zu erhöhen, ohne dass die Wundauflage abgenommen werden müsste. Durch die erfindungsgemäße Ausbildung mit mehreren entfernbaren Abschnitten kann die Länge der Auflageanordnung an die Länge beispielsweise eines Operationsschnitts angepasst werden.

Die Breite der Abschnitte muss nicht immer dieselbe sein, obwohl regelmäßig, insbesondere aus fertigungstechnischen Gründen, eine konstante Breite der Abschnitte zweckmäßig ist. Für bestimmte Anwendungsgebiete kann es aber auch sinnvoll sein, wenn die Breite der Abschnitte zu einem Ende hin oder zu beiden Enden hin abnimmt.

Die Längsrichtung der Auflageanordnung ist im Regelfall eine Gerade. Allerdings ist es nicht ausgeschlossen, dass die Auflageanordnung mit den Abschnitten eine leichte Krümmung ausbildet, wobei die Enden der Auflageanordnung zueinander in einem Winkel von einer 90°, vorzugsweise < 60° zueinander stehen. Die Abweichung von der Geraden, die durch ein Ende läuft, sollte am anderen Ende somit weniger als 90°, vorzugsweise weniger als 60°, betragen.

Die quer zur Längsrichtung ausgerichteten Soll-Trennlinien zwischen den Abschnitten liegen vorzugsweise rechtwinklig zur Längsrichtung. Dies ist jedoch nicht zwingend, da es nicht ausgeschlossen ist, dass das durch die Soll-Trennlinie gebildete jeweilige Ende des Abschnitts als Gerade schräg verläuft. Möglich ist ferner, dass die Soll-Trennlinie aus mehreren geradlinigen Abschnitten gebildet ist, die in einem Winkel zueinander stehen, sodass die Begrenzung des Abschnitts beispielsweise pfeilförmig ausgebildet sein kann. Ebenso ist es möglich, die Soll-Trennlinie in einer leichten Krümmung auszubilden, um ein abgerundetes Ende der Auflageanordnung zu ermöglichen.

In einer fertigungstechnisch bevorzugten Ausführungsform weisen die gleich aufgebauten Abschnitte jeweils eine gleiche Länge in Längsrichtung auf. Die Abschnitte können daher neben dem gleichen Aufbau auch eine gleiche Form und/oder Dimensionierung aufweisen.

Die im Dielektrikum eingebettete Elektrodenanordnung kann in an sich bekannter Weise durch wenigstens zwei voneinander isolierte Elektrodenflächen gebildet sein. Die Elektrodenflächen erstrecken sich als streifenförmige Flächen durch die Länge der Auflageanordnung hindurch. Dabei ist es möglich, dass die voneinander isolierten Elektrodenflächen mit demselben Hochspannungspotential beaufschlagt werden, sodass die zu behandelnde Oberfläche, auf der die Auflageanordnung aufliegt, als Gegenelektrode für die Plasmabildung fungiert. Gleiches gilt, wenn die beiden Elektrodenflächen mit gegenpoligen Hochspannungssignalen, insbesondere in Form von schnell abklingenden Wechselspannungspulsen, beaufschlagt werden und die zu behandelnde Oberfläche ein Mittenpotential (Massepotential) darstellt.

Alternativ ist es aber auch möglich, die beiden Elektroden als Elektrode und Gegenelektrode zu verwenden, sodass ein Plasmafeld nur oberflächlich zwischen den Elektrodenflächen ausgebildet wird.

In einer Ausführungsform weist das isolierende Bauelement, das die Soll-Trennlinie abdeckt, eine Verbindungsanordnung für wenigstens zwei voneinander isolierte Elektrodenflächen auf. Durch diese Verbindung ist es möglich zu überprüfen, ob das isolierende Bauelement zur Vermeidung einer direkten Zugänglichkeit der Elektroden an der Soll-Trennlinie ordnungsgemäß aufgebracht ist. Die Verbindung der beiden Elektrodenflächen kann beispielsweise durch eine Widerstandsmessanordnung leicht überprüft werden. Dadurch ist es möglich, dass eine Steuerung die Zuleitung von Hochspannungssignalen beziehungsweise -impulsen zu der Elektrodenanordnung nur dann freigibt, wenn das isolierende Bauelement die in die Endfläche der Soll-Trennlinie einmündenden Elektrodenflächen zuverlässig abdeckt.

In einer Ausführungsform der Erfindung ist die Auflageanordnung über ihre gesamte Länge durch die Abschnitte gebildet, besteht also ausschließlich aus den gleich aufgebauten Abschnitten. Die Zuführung der Hochspannungssignale zu der Elektrodenanordnung kann in diesem Fall günstig durch das isolierende Bauelement erfolgen, das eine Versorgungsanordnung zur Zuführung der Hochspannungssignale zu der Elektrodenanordnung enthält.

Die Versorgungsanordnung kann dabei eine bloße Kontaktierung der Elektrodenanordnung sein, wenn die Hochspannungssignale durch ein externes Gerät generiert und auf die Versorgungsanordnung geleitet werden.

In einer anderen Ausführungsform enthält die Versorgungsanordnung eine Steuerschaltung zur Generierung der Hochspannungssignale. In diesem Fall kann es ausreichend sein, der Versorgungsanordnung lediglich eine normale Versorgungsspannung, die keine Hochspannung sein muss, zuzuleiten.

Ferner ist es möglich, dass die Versorgungsanordnung neben der Steuerschaltung zur Generierung der Hochspannungssignale auch Batterien enthält, aus deren Spannung die Steuerschaltung die Hochspannungssignale generiert. In dieser Ausführungsform ist die Auflageanordnung autark und benötigt keinen externen Anschluss mehr. Die Versorgungsanordnung mit den Batterien ist dann entweder in dem Endabschnitt oder in dem isolierenden Bauelement realisiert.

In einer weiteren Ausführungsform können sich in den Abschnitten der Auflageanordnung Sensoren zur Abgabe wenigstens eines Sensorsignals für wenigstens einen an der Anlageseite messbaren Parameter befinden. Die Messung derartiger Parameter kann insbesondere bei der Anwendung auf der Haut als zu behandelnde Oberfläche sinnvoll sein. So ist es möglich, Hautrötungen, lokale Erwärmungen, Änderungen des pH-Werts der Haut usw. zu bestimmen. Im Falle einer Wundauflage können durch derartige Messungen lokale Infektionen früh erkannt und dadurch eine Plasmabehandlung initiiert werden, die aufgrund der keimreduzierenden Wirkung der Infektion entgegenwirkt.

Eine Auswertungsanordnung für die Sensorsignale kann in der Versorgungsanordnung enthalten sein. Alternativ ist es aber auch möglich, die Sensorsignale direkt am Sensor zu erkennen oder vom Sensor mit einem Übertragungskabel oder drahtlos auf ein entsprechendes Empfangsgerät zu übertragen. Die drahtlose Übertragung erfolgt vorteilhaft in einem Nahkommunikationssystem, beispielsweise nach dem Bluetooth-Standard. Eine Auswertungseinrichtung dient der Auswertung der Sensorsignale und ggf. einer Alarmgabe. Ferner kann die Behandlung mit dem Plasma durch Auswertung der Sensorsignale gesteuert werden.

Das Abtrennen einzelner Abschnitte der Auflageanordnung kann dadurch unterstützt werden, dass das Dielektrikum auf der Soll-Trennlinie das Abtrennen erleichternde Materialschwächungen aufweist. Die Materialschwächungen können Einkerbungen und andere Vertiefungen, aber auch Durchgangslöcher sein. Durchgangslöcher dürfen jedoch nur in den Bereichen des Dielektrikums vorgesehen sein, in denen sich keine Elektrodenfläche befindet, um nicht das Risiko eines direkten Stromflusses von der mit Hochspannung versorgten Elektrode einzugehen.

Grundsätzlich ist es bekannt, dass sowohl das Dielektrikum als auch die Elektrodenanordnung zur Anpassung der Anlageseite an Unebenheiten der zu behandelnden Oberfläche, auf die die Auflageanordnung aufgelegt wird, flexibel auszubilden. Insbesondere zur Behandlung der Haut, einschließlich von im Hautbereich befindlichen Wunden, ist die flexible Ausbildung der Auflageanordnung zweckmäßig.

Die erfindungsgemäße Auflageanordnung kann ferner in an sich bekannter Weise mit zahlreichen Durchgangsöffnungen versehen sein, die im Dielektrikum von der Auflageseite zu einer Oberseite hin durchgehend ausgebildet sind, wobei die Elektrodenanordnung nicht in dem Bereich der Durchgangsöffnungen hineinragt, sodass die Durchgangsöffnungen durchgehende Kanäle mit ununterbrochenen Dielektrikumswandungen ausbilden, sodass auch in diesem Bereich ein direkter Stromfluss von der Elektrodenanordnung ausgehend ausgeschlossen ist. Die Erfindung soll im Folgenden anhand von in der Zeichnung dargestellten Ausführungsbeispielen näher erläutert werden. Es zeigen:
- Figur 1: eine erste Ausführungsform einer Auflageanordnung mit einer Ansicht von oben, einer Ansicht von unten (Anlageseite), einer Horizontalschnittdarstellung und einer Vertikalschnittdarstellung;
- Figur 2: eine zweite Ausführungsform in den gleichen Darstellungen wie Figur 1 ;
- Figur 2A: vergrößerte Darstellungen von drei Abschnitten des Vertikalschnitts gemäß Fig. 2d);
- Figur 3: eine dritte Ausführungsform in den gleichen Darstellungen wie Figur 1 ;
- Figur 4: eine vierte Ausführungsform mit einer Ansicht von unten, einer Horizontalschnittdarstellung und einer Vertikalschnittdarstellung;
- Figur 5: eine fünfte Ausführungsform in den Darstellungen wie Figur 1 ;
- Figur 6: eine sechste Ausführungsform in den Darstellungen wie Figur 1.

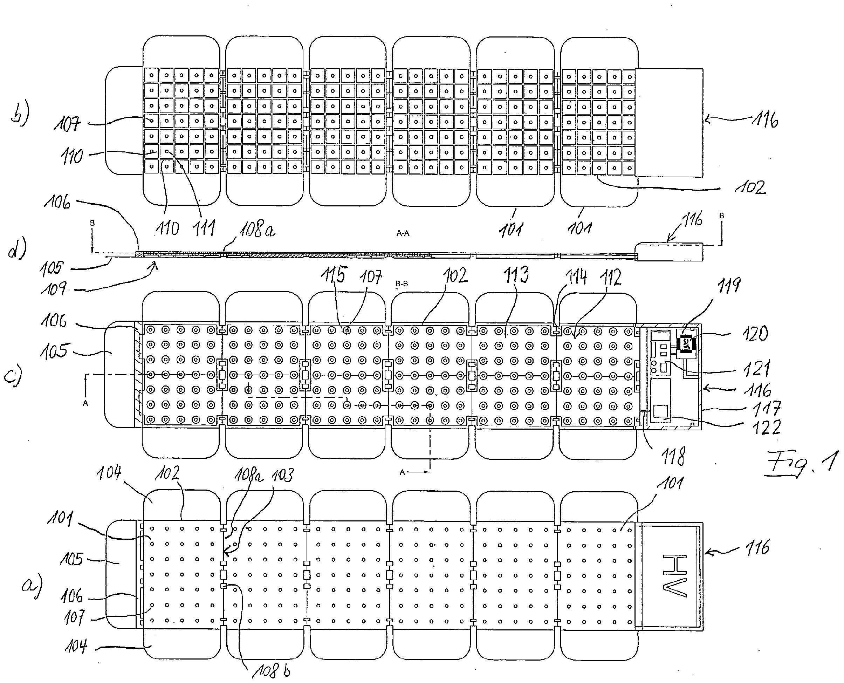

Die in Figur 1 dargestellte erste Ausführungsform lässt in der Draufsicht der Figur 1 a) erkennen, dass die Auflageanordnung aus sechs im wesentlichen gleichen Abschnitten 101 besteht, die jeweils an der Oberseite ein Dielektrikum 102 aufweisen, das in dem dargestellten Ausführungsbeispiel in der Draufsicht rechteckig ausgebildet ist. Die Dielektrika 102 der Abschnitte 101 weisen alle eine gleiche Breite auf und sind in einer Längsrichtung L über Soll-Trennlinien 103 aneinander angeschlossen. Die Soll-Trennlinien 103 laufen senkrecht zu der Längsrichtung L.

In Breitenrichtung sind an die Dielektrika 102 jedes Abschnitts 101 beidseitig jeweils ein folienartiger Flügel 104 angeformt, der auf seiner Unterseite mit einem Haftklebstoff beschichtet ist und zur Befestigung der Auflageanordnung auf einer zu behandelnden Oberfläche, insbesondere auf der Haut eines Menschen, dient. Der sich in Figur 1 am linken Ende befindliche Abschnitt 101 weist zusätzlich einen weiteren folienförmigen Flügel 105 auf, der sich somit an diesen Abschnitt 101 in Längsrichtung L anschließt. Zur Verbesserung der Abschirmung ist der Flügel 105 an das Dielektrikum 102 des Abschnitts 101 über einen Isoliersteg 106 angeschlossen. Das Dielektrikum 102 aller Abschnitte 101 bildet mit den Flügeln 104, 105 und dem Isoliersteg 106 eine einstückige Ausbildung aus demselben Material. Das Dielektrikum 102 jedes Abschnitts 101 weist eine Vielzahl von regelmäßig angeordneten Durchgangs-löchern 107 auf, durch die bei einer Verwendung der Auflageanordnung als Wundauflage Wundsekret abgesaugt werden kann. In anderen Anwendungsfällen ist es möglich, durch die Durchgangsöffnungen 107 heilende oder pflegende Mittel in flüssiger oder in Gasform in den Bereich der zu behandelnden Oberfläche einzuleiten.

Die Soll-Trennlinie 103 weist Materialschwächungen 108 in Form einer Ritzlinie 108a und in Form von Durchgangsöffnungen 108b auf. Durch diese Materialschwächung-en ist es bei geeigneten Materialien für das Dielektrikum 102 möglich, die Abtrennung eines Abschnitts 101 entlang der Soll-Trennlinie 103 durch Abreißen, d. h. ohne Werkzeug, durchzuführen. Aber auch, wenn für das Abtrennen beispielsweise eine Schere benutzt wird, sind die vorgesehenen Materialschwächungen hilfreich, um die Abtrennung entlang der vorgesehen Soll-Trennlinie 103 durchzuführen.

Figur 1 b) zeigt eine Ansicht von unten, also von einer Anlageseite 109 der Auflageanordnung. Auf der Anlageseite 109 ist das Dielektrikum in Form von ein gleichmäßiges Gitter formenden, senkrecht zueinander stehenden Stegen 110 ausgebildet. Die Stege 110 sind gleich hoch geformt, bilden mit ihren freien (unteren) Kanten eine Anlagefläche und begrenzen seitlich eine nach unten, d. h. zur Anlageseite 109 hin offene Kammer 111. Die Kammer 111 wird nach oben hin durch eine durchgehende Schicht des Dielektrikums 102 begrenzt, in der sich mittig in jeder Kammer 111 die Durchgangsöffnung 107 befindet. Die Kammern 111 können leer bleiben oder beispielsweise mit heilenden und / oder pflegenden Substanzen teilweise befüllt sein.

Die Stege 110 bilden somit einen Abstandshalter zur Ausbildung eines Luftvolumens innerhalb der Kammern 111, in dem sich das Plasma zur Behandlung der Oberfläche, insbesondere zur Behandlung der Haut, ausbilden kann.

Figur 1 c) zeigt einen Horizontalschnitt entlang der Linie B-B, wie sie in Figur 1 d) eingezeichnet ist. Der Horizontalschnitt ermöglicht eine Draufsicht auf eine Elektrodenanordnung 112, die allseitig vom Material des Dielektrikums 102 umgeben ist. Die in Figur 1 dargestellte Elektrodenanordnung 112 ist eine flächige leitende Schicht, die eine einzige zusammenhängende Elektrodenfläche 113 als eine einzige Elektrode ausbildet. Die Fläche weist Ausnehmungen 114 im Bereich der als Durchgangsöffnungen 108b ausgebildeten Materialschwächungen 108 auf. Ferner ist erkennbar, dass die Elektrodenfläche 113 im Bereich der Durchgangsöffnungen 107 ebenfalls Durchgangsöffnungen 115 aufweist, deren Durchmesser jedoch größer ist als der Durchmesser der Durchgangsöffnungen 107 des Dielektrikums 102. Demgemäß erstreckt sich die Elektrodenfläche 113 nicht bis in den Bereich der Wandung der Durchgangsöffnung 107 hinein. Die Durchgangsöffnung wird somit von einer Wandung begrenzt, die ununterbrochen aus dem Material des Dielektrikums 102 besteht.

Figur 1 d) zeigt einen Hochschnitt, der die Ausbildung des Dielektrikums 102 ebenfalls verdeutlicht. Der Verlauf des in Figur 1 d) dargestellten Vertikalschnitts ist in Figur 1 c) durch die Schnittlinie A-A verdeutlicht.

Die aus den Abschnitten 101 gebildete Auflageanordnung wird in dem Ausführungsbeispiel gemäß Figur 1 komplettiert durch ein isolierendes Bauelement 116, das als isolierendes Gehäuse die Soll-Trennlinie 103 des in Figur 1 ganz rechts dargestellten letzten Abschnitts 101 übergreift, sodass die in die Stirnfläche der Soll-Trennlinie 103 des Abschnitts 101 hineinragende Elektrodenfläche 113 von dem isolierenden Bauelement 116 sicher isolierend abgedeckt wird. Das isolierende Bauelement 116 enthält in dieser Ausführungsform eine Versorgungsanordnung 117, über die Hochspannungssignale über eine Zuleitung 118 auf die Elektrodenanordnung 112 übertragen werden. Die Versorgungsanordnung 117 enthält hierbei eine Steuerung 119 mit einem Mikrocontroller 120, einer Signalformungsstufe 121 und einer Transformatorstufe 122 zur Ausbildung der Hochspannung. In dieser Ausführungsform wird der Versorgungsanordnung 117 eine Versorgungsspannung von außen zugeführt, die eine Wechselspannung oder eine Gleichspannung sein kann.

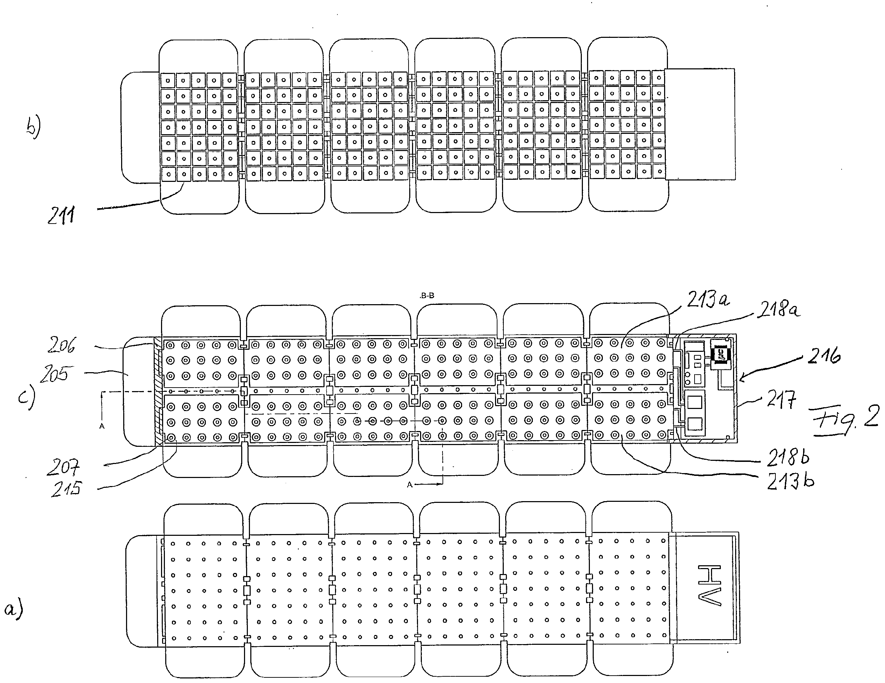

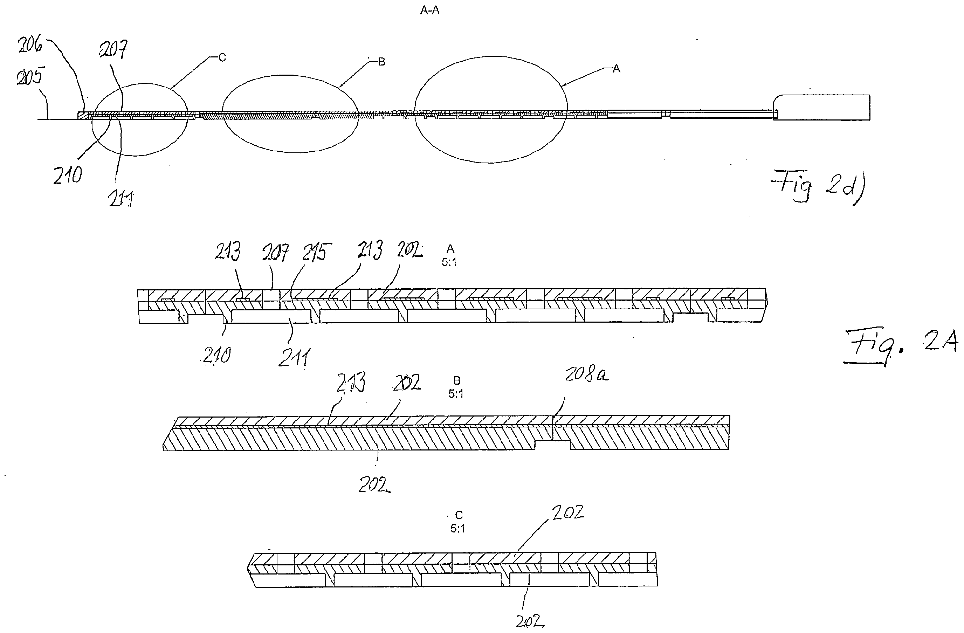

Die in Figur 2 dargestellte Ausführungsform entspricht der Ausführungsform gemäß Figur 1 weitgehend, sodass im Folgenden nur die Unterschiede erläutert werden. Die für die Ausführungsform gemäß Figur 1 verwendeten Bezugsziffern 1xx werden für die weitere Ausführungsform als 2xx mit dem identischen Bestandteil xx beibehalten. In analoger Weise werden die Bezugsziffern für die weiteren Ausführungsformen verwendet. In der Ausführungsform gemäß Figur 2 besteht die Elektrodenanordnung 212 aus zwei voneinander isolierten Elektrodenflächen 213a, 213b, die sich jeweils über die Länge der Auflageanordnung erstrecken. Die Versorgungsanordnung 217 in dem isolierenden Bauelement 216 enthält eine Transformatorstufe 122 mit zwei Transformatoren, die über Zuleitungen 218a und 218b, die beiden Elektrodenflächen 213a und 213b jeweils mit Hochspannungssignalen versorgen. Die Hochspannungssignale weisen dabei eine identische Form auf, sind jedoch zueinander umgekehrt polarisiert, sodass beispielsweise die Elektrodenfläche 213a mit einem positiven Hochspannungsimpuls und gleichzeitig die Elektrodenfläche 213b mit einem negativen Hochspannungsimpuls der gleichen Größe versorgt wird. Auf diese Weise entsteht zwischen den Elektrodenflächen auf der Anlageseite 209 ein verstärktes Differenzfeld, obwohl für beide Hochspannungsimpulse die zu behandelnde Oberfläche als Masseelektrode wirkt. Auf diese Weise wird die Effizienz für die Ausbildung eines Plasmas in den Kammern 211 verbessert.

Figur 2A enthält den Hochschnitt gemäß Figur 2d) mit Markierungen von Detail-Abschnitten, die als Detail A, B und C im Maßstab 5:1 dargestellt sind. Der Hochschnitt verläuft entlang der in Figur 2c) eingezeichneten Schnittlinie A-A.

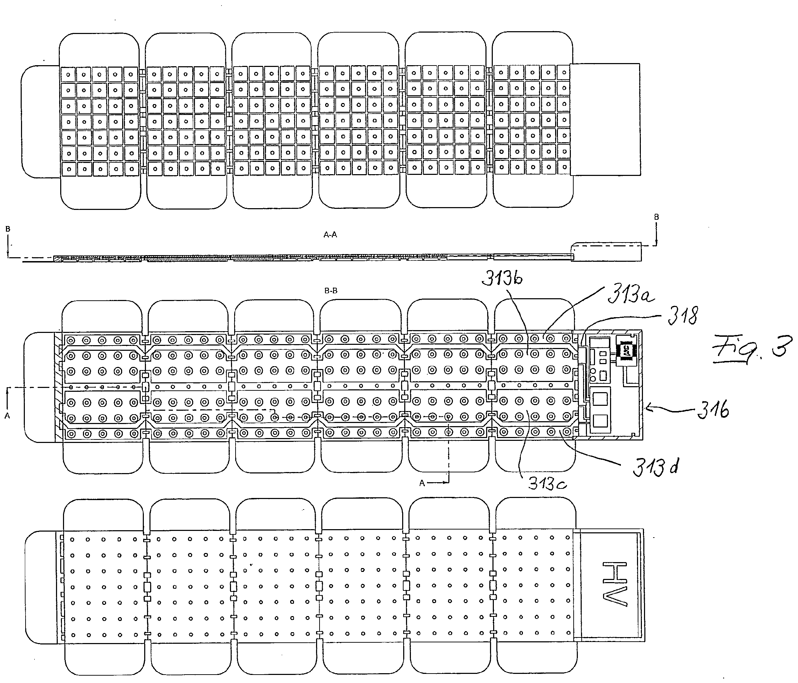

Bei dem in Figur 3 dargestellten Ausführungsbeispiel, das weitgehend dem Ausführungsbeispiel gemäß Figur 2 entspricht, sind vier Elektrodenflächen 313a, 313b, 313c, 313d vorgesehen, die voneinander isoliert sind und sich jeweils über die Länge der Auflageanordnung erstrecken. In diesem Fall werden die Elektrodenflächen 313a und 313b mit den Hochspannungssignalen der einen Polung und die Elektrodenflächen 313c und 313d mit den Hochspannungssignalen der umgekehrten Polarität über die jeweiligen Zuleitungen 318 versorgt. Auch in dieser Anordnung wird die zu behandelnde Oberfläche als Gegenelektrode genutzt.

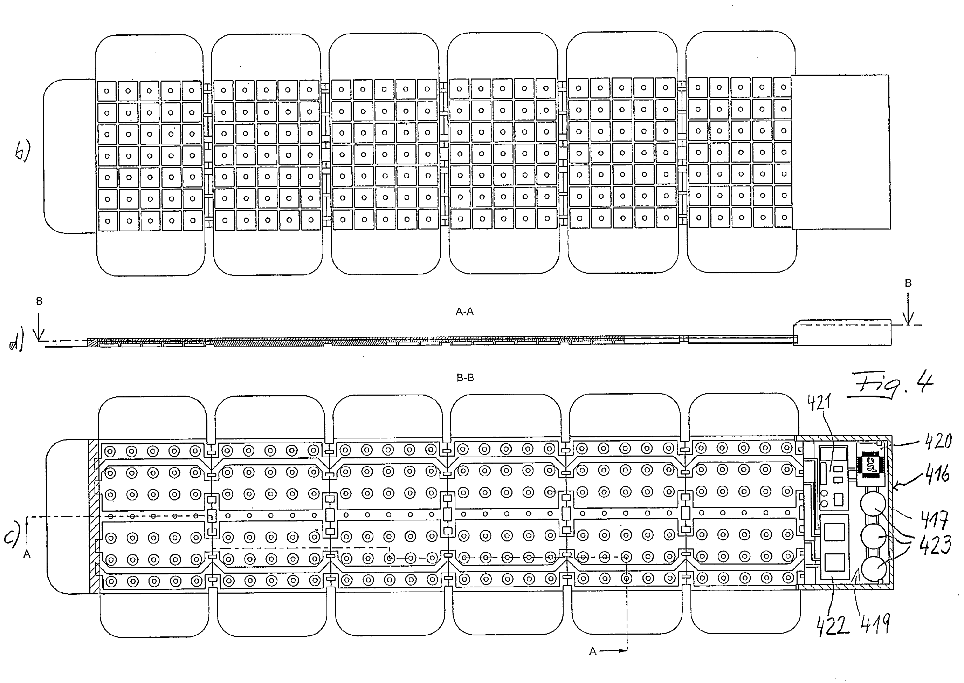

Bei der in Figur 4 dargestellten Ausführungsform ist auf die gleich aussehende Darstellung der Draufsicht von oben verzichtet worden, sodass nur die Darstellungen 4 b), 4 c) und 4 d) vorhanden sind. Die Ausführungsform entspricht der Ausführungsform gemäß Figur 3. Im Unterschied zu der in Figur 3 dargestellten Ausführungsform weist die Versorgungsanordnung 417 eine Steuerung 419 auf, die neben dem Mikrocontroller 420, der Signalformungsstufe 421 und der Transformatorstufe 422 noch drei Batterien 423 enthält. Durch die Batterien 423 können die übrigen Bauelemente der Steuerung 419 die für die Ausbildung der Hochspannungssignale benötigte Energie autark beziehen, sodass keine Zuleitung von Energie von außen erforderlich ist.

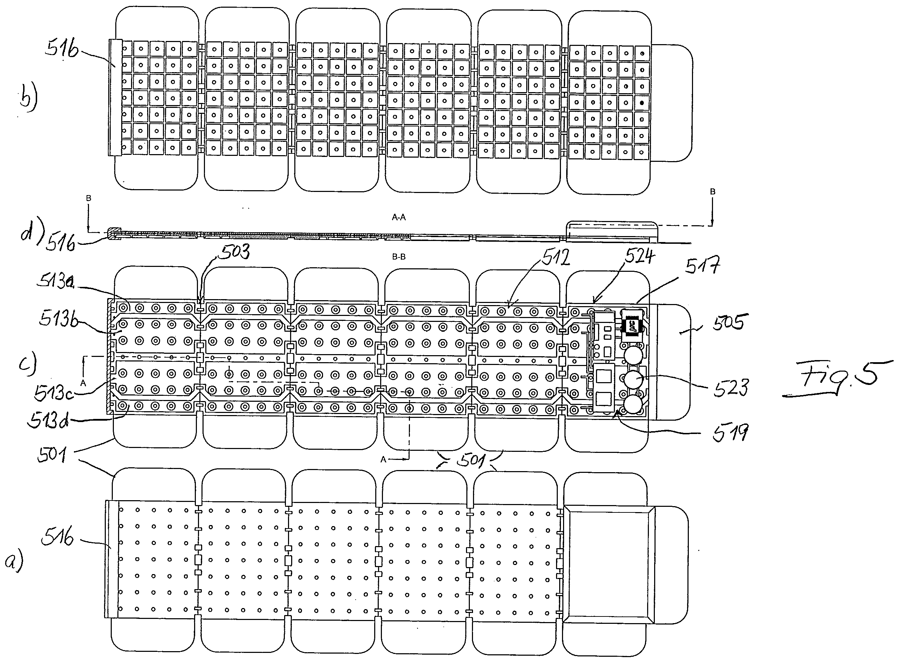

Das in Figur 5 dargestellte fünfte Ausführungsbeispiel entspricht funktionsmäßig dem in Figur 4 dargestellten vierten Ausführungsbeispiel. Die Versorgungsanordnung 517 befindet sich im Unterscheid zu dem vierten Ausführungsbeispiel gemäß Figur 4 hier jedoch nicht in dem isolierenden Bauelement, sondern in einem Endabschnitt 524, der in diesem Ausführungsbeispiel die gleiche Größe wie die Abschnitte 501 aufweist, jedoch mit der Versorgungsanordnung 517 und der Steuerung 519 mit den Batterien 523 versehen ist. In diesem Fall befindet sich der in Längsrichtung angeordnete Flügel 505 an dem Endabschnitt 524. Die mögliche Abtrennung von Abschnitten 501 erfolgt somit vom anderen Ende her. Die sich in die Endfläche der Soll-Trennlinien 503 des letzten Abschnitts 501 erstreckenden Elektrodenflächen 513a, 513b, 513c und 513d werden hier durch ein schmales isolierendes Bauelement 516 abgedeckt, das mit einer (nicht dargestellten) Verbindungsanordnung wenigstens zwei der Elektrodenflächen 513a, 513b, 513c, 513d miteinander verbindet, woraus die Steuerung 519 ein Signal generiert, das die ordnungsgemäße Anordnung des isolierenden Bauelements 516 am Ende der Auflageanordnung charakterisiert und somit als Initialisierungssignal für die Generierung der Hochspannungssignale dienen kann. Die Hochspannungssignale für die Elektrodenflächen 513a, 513b, 513c, 513d werden somit erst generiert, wenn sichergestellt ist, dass die freie Soll-Trennlinie 503 am Ende des letzten Abschnitts 501 ordnungsgemäß durch das isolierende Bauelement 516 abgedeckt ist.

Figur 5 d) lässt erkennen, dass die Steuerung 519 in dem Endabschnitt 524 in einer vergrößerten Materialansammlung des Dielektrikums 502 eingebettet, vorzugsweise eingegossen, ist.

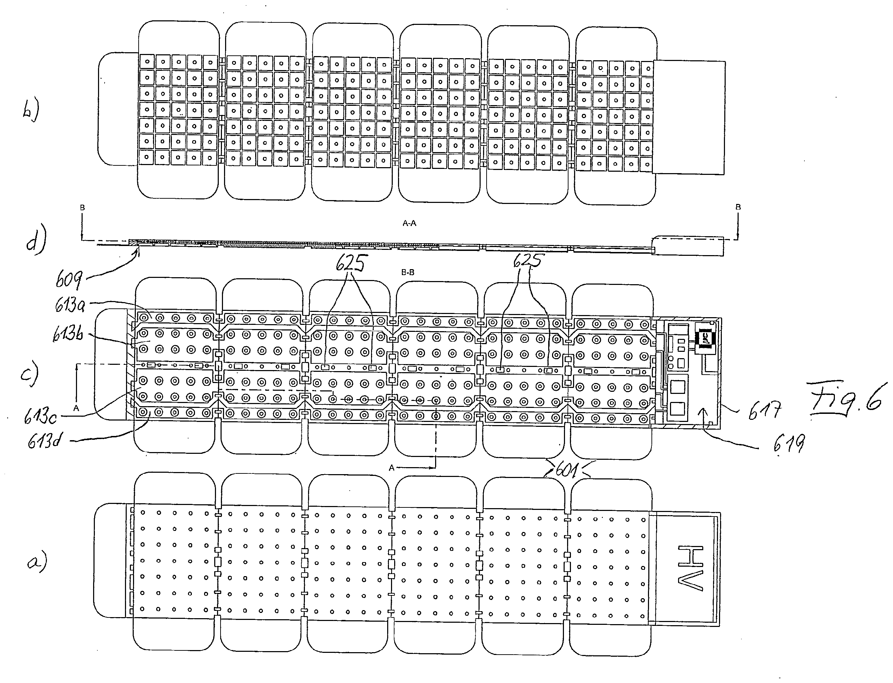

Die in Figur 6 dargestellte sechste Ausführungsform entspricht der in Figur 3 dargestellten Ausführungsform, weist jedoch innerhalb eines die Elektrodenflächen 613b und 613c voneinander isolierenden dielektrischen Streifens Sensoren 625 auf, mit denen Parameter der zu behandelnden Oberfläche, insbesondere einer Hautoberfläche oder einer Wundfläche, gemessen und angezeigt bzw. auf den Mikrocontroller 620 in der Steuerung 619 übertragen werden können. So ist es beispielsweise möglich, berührungslos die Sauerstoffsättigung des Blutes in der Hautschicht zu messen und die Plasmabehandlung zu initiieren, wenn die Sauerstoffsättigung einen Schwellwert unterschreitet. In ähnlicher Weise können andere Parameter, wie Temperatur, Rötung aufgrund eines Infekts, pH-Wert usw. festgestellt und ausgewertet und ggf. angezeigt werden.

Die Sensoren können dabei selbst als Anzeigeelement dienen, wenn sie aufgrund einer physikalischen oder chemischen Reaktion eine Anzeige für eine Änderungen oder Überschreitung eines Messparameters verdeutlichen. Die Sensorreaktion kann in diesem Fall unmittelbar optisch wahrgenommen oder auch elektrisch auf eine Auswertungseinrichtung übertragen werden. Alternativ dazu sind Sensoren 625 einsetzbar, die für ihre Funktion und / oder Auswertung einer Spannungsversorgung bedürfen.

Die Sensoren 625 können von der Versorgungsanordnung 617 aus, wenn erforderlich, mit einer Versorgungsspannung versorgt werden und so ausgebildet sein, dass sie die Sensorsignale auch über eine Nahkommunikation (beispielsweise nach dem Bluetooth-Standard) drahtlos auf eine Auswertungsstufe übertragen.

In allen Ausführungsformen wird das Dielektrikum 102, 202,... durch einen isolierenden Kunststoff, insbesondere gießfähiges Silikon, gebildet. Die Elektrodenflächen 113, 213,... werden vorzugsweise ebenfalls durch einen gießfähigen Kunststoff gebildet, der mit dem Material des Dielektrikums 102, 202,... verträglich ist, also insbesondere auch ein Silikon sein kann. Die Eigenschaft als leitende Elektrodenfläche wird dabei durch leitende Zusätze zu dem an sich nicht leitenden Trägerkunststoff realisiert. Selbstverständlich ist es aber auch möglich, die Elektrodenflächen durch eine metallisch leitende Folie zu verwirklichen.

## Patentansprüche

1. Flächige Auflageanordnung, ausgebildet zur Erzeugung eines dielektrisch behinderten Plasmas an einer Anlageseite (109) der Auflageanordnung, mit einer flächigen Elektrodenanordnung (112), die in ein flächiges Dielektrikum (102) eingebettet, mit Hochspannungssignalen versorgbar und allseitig gegen einen unbehinderten Stromfluss abgeschirmt ist, wobei sich die Auflageanordnung mit einer Breite in einer Längsrichtung (L) erstreckt und wobei die Länge der Auflageanordnung in dieser Längsrichtung verkleinerbar ist, **dadurch gekennzeichnet, dass** die Auflageanordnung in Längsrichtung (L) mehrere gleich aufgebaute Abschnitte (101) mit jeweils einem Dielektrikumsabschnitt in der Breite der Auflageanordnung und mit jeweils wenigstens einem Elektrodenabschnitt aufweist, wobei die Elektrodenabschnitte der Abschnitte (101) in Längsrichtung (L) aneinander anschließen und eine sich über die gesamte Länge erstreckende Elektrodenanordnung (112) bilden, dass wenigstens ein Abschnitt (101) von einem benachbarten Abschnitt (101) zur Verkleinerung der Anlagefläche in Längsrichtung (L) an einer quer zur Längsrichtung (L) verlaufenden Soll-Trennlinie (103) abtrennbar ist und dass zum Abdecken der Soll-Trennlinie (103) an dem verbleibenden Abschnitt (101) ein isolierendes Bauelement (116) vorgesehen ist.

2. Auflageanordnung nach Anspruch 1, **dadurch gekennzeichnet, dass** die gleich aufgebauten Abschnitte (101) eine gleiche Länge in Längsrichtung (L) aufweisen.

3. Auflageanordnung nach Anspruch 1 oder 2, **dadurch gekennzeichnet, dass** die Elektrodenanordnung (112) wenigstens zwei voneinander isolierte Elektrodenflächen (113) aufweist.

4. Auflageanordnung nach Anspruch 3, **dadurch gekennzeichnet, dass** das isolierende Bauelement (516) eine Verbindungsanordnung für wenigstens zwei voneinander isolierte Elektrodenflächen (513a bis 513d) aufweist.

5. Auflageanordnung nach einem der Ansprüche 1 bis 4, **dadurch gekennzeichnet, dass** sie über ihre gesamte Länge durch die Abschnitte (101) gebildet ist.

6. Auflageanordnung nach einem der Ansprüche 1 bis 5, **dadurch gekennzeichnet, dass** das isolierende Bauelement (116) eine Versorgungsanordnung (117) zur Zuführung von Hochspannungssignalen zu der Elektrodenanordnung (112) enthält.

7. Auflageanordnung nach einem der Ansprüche 1 bis 4, **dadurch gekennzeichnet, dass** sie über ihre gesamte Länge mit Ausnahme eines Endabschnitts (524) durch die Abschnitte (501) gebildet ist und dass der Endabschnitt (524) eine Versorgungsanordnung (517) zur Zuführung von Hochspannungssignalen zu der Elektrodenanordnung (512) enthält.

8. Auflageanordnung nach einem der Ansprüche 5 bis 7, **dadurch gekennzeichnet, dass** eine Versorgungsanordnung (117) zur Zuführung von Hochspannungssignalen zu der Elektrodenanordnung (112) eine Steuerschaltung (119) zur Generierung der Hochspannungssignale aufweist.

9. Auflageanordnung nach Anspruch 8, **dadurch gekennzeichnet, dass** die Versorgungsanordnung (417) ferner Batterien (423) enthält, aus deren Spannung die Steuerschaltung (419) die Hochspannungssingale generiert.

10. Auflageanordnung nach einem der Ansprüche 1 bis 9, **dadurch gekennzeichnet, dass** in den Abschnitten (601) Sensoren (625) zur Abgabe wenigstens eines Sensorsignals für wenigstens einen an der Anlageseite (609) messbaren Parameter vorhanden sind.

11. Auflageanordnung nach Anspruch 10, **dadurch gekennzeichnet, dass** eine Auswertungsanordnung für die Sensorsignale einer Versorgungsanordnung (617) zur Zuführung von Hochspannungssignalen zu der Elektrodenanordnung (112) enthalten ist.

12. Auflageanordnung nach einem der Ansprüche 1 bis 11, **dadurch gekennzeichnet, dass** das Dielektrikum (102) auf der Soll-Trennlinie (103) das Abtrennen erleichternde Materialschwächungen (108) aufweist.

13. Auflageanordnung nach Anspruch 12, **dadurch gekennzeichnet, dass** die Materialschwächungen (108) zumindest teilweise Durchgangslöcher (108b) in Bereichen des Dielektrikums (102) sind, in denen sich keine Elektrodenfläche (113) befinden.

14. Auflageanordnung nach einem der Ansprüche 1 bis 13, **dadurch gekennzeichnet, dass** sowohl das Dielektrikum (102) als auch die Elektrodenanordnung (112) zur Anpassung der Anlageseite (109) an Unebenheiten flexibel ausgebildet sind.

15. Auflageanordnung nach einem der Ansprüche 1 bis 14, **dadurch gekennzeichnet, dass** die Breite des Dielektrikums (102) über die Länge der Auflageanordnung konstant ist.

## Claims

1. A planar contact arrangement, formed for generating a dielectric barrier plasma on an application side (109) of the contact arrangement, having a planar electrode arrangement (112), which is embedded in a planar dielectric material (102), can be supplied with high-voltage signals, and is shielded on all sides against an unobstructed current flow, wherein the contact arrangement extends with a width in a longitudinal direction (L) and wherein the length of the contact arrangement is resizable in this longitudinal direction (L), **characterized in that** the contact arrangement comprises multiple identically constructed sections (101) in the longitudinal direction (L) each having a dielectric section in the width of the contact arrangement and each having at least one electrode section, wherein the electrode sections of the sections (101) adjoin one another in the longitudinal direction (L) and form an electrode arrangement (112) extending over the entire length, **in that** at least one section (101) is separable at an intended separation line (103) extending transversely to the longitudinal direction (L) from an adjacent section (101) to reduce the size of the application area in the longitudinal direction (L), and **in that** an insulating component (116) is provided to cover the intended separation line (103) on the remaining section (101).

2. The contact arrangement as claimed in claim 1, **characterized in that** the identically constructed sections (101) have an equal length in the longitudinal direction (L).

3. The contact arrangement as claimed in claim 1 or 2, **characterized in that** the electrode arrangement (112) comprises at least two electrode areas (113) insulated from one another.

4. The contact arrangement as claimed in claim 3, **characterized in that** the insulating component (516) comprises a connection arrangement for at least two electrode areas (513a to 513d) insulated from one another.

5. The contact arrangement as claimed in any one of claims 1 to 4, **characterized in that** it is formed over its entire length by the sections (101).

6. The contact arrangement as claimed in any one of claims 1 to 5, **characterized in that** the insulating component (116) contains a supply arrangement (117) for supplying high-voltage signals to the electrode arrangement (112).

7. The contact arrangement as claimed in any one of claims 1 to 4, **characterized in that** it is formed over its entire length with the exception of an end section (524) by the sections (501), and **in that** the end section (524) contains a supply arrangement (517) for supplying high-voltage signals to the electrode arrangement (512).

8. The contact arrangement as claimed in any one of claims 5 to 7, **characterized in that** the supply arrangement (117) comprises a control circuit (119) for generating the high-voltage signals.

9. The contact arrangement as claimed in claim 8, **characterized in that** the supply arrangement (417) furthermore contains batteries (423), from the voltage of which the control circuit (419) generates the high-voltage signals.

10. The contact arrangement as claimed in any one of claims 1 to 9, **characterized in that** sensors (625) for emitting at least one sensor signal for at least one parameter measurable on the application side (609) are provided in the sections (601).

11. The contact arrangement as claimed in claim 10, **characterized in that** an evaluation arrangement for the sensor signals is contained in the supply arrangement (617).

12. The contact arrangement as claimed in any one of claims 1 to 11, **characterized in that** the dielectric material (102) has weakened material areas (108), which facilitate the separation, on the intended separation line (103).

13. The contact arrangement as claimed in claim 12, **characterized in that** the weakened material areas (108) are at least partially passage holes (108b) in the region of the dielectric material (102), in which an electrode area (113) is not located.

14. The contact arrangement as claimed in any one of claims 1 to 13, **characterized in that** both the dielectric material (102) and also the electrode arrangement (112) are formed flexible for adaptation of the application side (109) to irregularities.

15. The contact arrangement as claimed in any one of claims 1 to 14, **characterized in that** the width of the dielectric material (102) is constant over the length of the contact arrangement.

## Revendications

1. Ensemble de support plat, conçu pour générer un plasma à barrière diélectrique sur une face d'appui (109) de l'ensemble de support, comportant un ensemble d'électrode plat (112) qui est noyé dans un diélectrique plat (102), qui peut être alimenté en signaux haute tension et qui est protégé de tous côtés contre un flux de courant non entravé, l'ensemble de support s'étendant avec une largeur dans une direction longitudinale (L), et la longueur de l'ensemble de support pouvant être réduite dans ladite direction longitudinale,
**caractérisé en ce que**
l'ensemble de support présente, dans la direction longitudinale (L), plusieurs portions (101) de construction identique, présentant chacune une portion diélectrique dans la largeur de l'ensemble de support et présentant chacune au moins une portion d'électrode, les portions d'électrode desdites portions (101) se raccordant les unes aux autres dans la direction longitudinale (L) et formant un ensemble d'électrode (112) qui s'étend sur toute la longueur,
**en ce qu'**au moins une portion (101) peut être séparée d'une portion adjacente (101) le long d'une ligne destinée à la séparation (103) qui s'étend transversalement à la direction longitudinale (L), afin de réduire la surface d'appui dans la direction longitudinale (L), et
**en ce qu'**un composant isolant (116) est prévu sur la portion restante (101), afin de recouvrir la ligne destinée à la séparation (103).

2. Ensemble de support selon la revendication 1,
**caractérisé en ce que** les portions (101) de construction identique ont la même longueur dans la direction longitudinale (L).

3. Ensemble de support selon la revendication 1 ou 2,
**caractérisé en ce que** l'ensemble d'électrode (112) présente au moins deux surfaces d'électrode (113) isolées l'une de l'autre.

4. Ensemble de support selon la revendication 3,
**caractérisé en ce que** le composant isolant (516) comprend un ensemble de connexion pour au moins deux surfaces d'électrode (513a à 513d) isolées l'une de l'autre.

5. Ensemble de support selon l'une des revendications 1 à 4,
**caractérisé en ce qu'**il est formé par lesdites portions (101) sur toute sa longueur.

6. Ensemble de support selon l'une des revendications 1 à 5,
**caractérisé en ce que** le composant isolant (116) comprend un ensemble d'alimentation (117) pour fournir des signaux haute tension à l'ensemble d'électrode (112).

7. Ensemble de support selon l'une des revendications 1 à 4,
**caractérisé en ce qu'**il est formé par lesdites portions (501) sur toute sa longueur, à l'exception d'une portion d'extrémité (524), et
**en ce que** ladite portion d'extrémité (524) comprend un ensemble d'alimentation (517) pour fournir des signaux haute tension à l'ensemble d'électrode (512).

8. Ensemble de support selon l'une des revendications 5 à 7,
**caractérisé en ce qu'**un ensemble d'alimentation (117) destiné à fournir des signaux haute tension à l'ensemble d'électrode (112) comprend un circuit de commande (119) pour générer les signaux haute tension.

9. Ensemble de support selon la revendication 8,
**caractérisé en ce que** l'ensemble d'alimentation (417) comprend en outre des piles (423), à partir de la tension desquelles le circuit de commande (419) génère les signaux haute tension.

10. Ensemble de support selon l'une des revendications 1 à 9,
**caractérisé en ce que** des capteurs (625) sont présents dans les portions (601) pour émettre au moins un signal de capteur pour au moins un paramètre qui peut être mesuré sur la face d'appui (609).

11. Ensemble de support selon la revendication 10,
**caractérisé en ce qu'**il est prévu un ensemble d'évaluation des signaux de capteur dans un ensemble d'alimentation (617) pour fournir des signaux haute tension à l'ensemble d'électrode (112).

12. Ensemble de support selon l'une des revendications 1 à 11,
**caractérisé en ce que** le diélectrique (102) présente des affaiblissements de matériau (108) sur la ligne destinée à la séparation (103), qui facilitent la séparation.

13. Ensemble de support selon la revendication 12,
**caractérisé en ce que** les affaiblissements de matériau (108) sont au moins partiellement des trous traversants (108b) dans des zones du diélectrique (102) dans lesquelles il n'y a pas de surface d'électrode (113).

14. Ensemble de support selon l'une des revendications 1 à 13,
**caractérisé en ce que** tant le diélectrique (102) que l'ensemble d'électrode (112) sont réalisés de façon flexible afin d'adapter la face d'appui (109) aux irrégularités.

15. Ensemble de support selon l'une des revendications 1 à 14,
**caractérisé en ce que** la largeur du diélectrique (102) est constante sur la longueur de l'ensemble de support.
